# EUROPEAN PATENT APPLICATION

(11) **EP 4 227 347 A1**
(43) Date of publication of application: **16.08.2023**
(21) Application number: 21877671.4
(22) Date of filing: 06.10.2021
(51) Int. Cl.: C08G 77/38, C08G 77/50, A61Q 1/00, C08L 83/10, C08L 83/14, C09K 3/18, A61K 8/891, C08K 3/22

(54) **CARBOXYLIC ACID-COMODIFIED ORGANOPOLYSILOXANE AND USE THEREFOR**

(30) Priority: 09.10.2020 JP 2020171043
(71) Applicant: Dow Toray Co., Ltd., Tokyo 140-8617 (JP)
(72) Inventor: PHAN SON THANH, Ichihara-shi Chiba 299-0108 (JP); KANZAKI Yasue, Ichihara-shi Chiba 299-0108 (JP); KIKUNAGA Sayuri, Ichihara-shi Chiba 299-0108 (JP); HORI Seiji, Ichihara-shi Chiba 299-0108 (JP)
(74) Representative: Murgitroyd & Company
(86) International application number: PCT/JP2021/037020
(87) International publication number: WO 2022/075372

(57) **Abstract**

[PROBLEM]

To provide a new carboxylic acid co-modified organopolysiloxane that can stably and uniformly disperse various hydrophobic powders in an aqueous phase and has excellent function as a powder dispersantor and the like, use thereof as a powder treatment agent, a topical agent composition, and the like containing this composition.

[RESOLUTION MEANS]

A novel carboxylic acid co-modified organopolysiloxane expressed by the following structural formula (1): 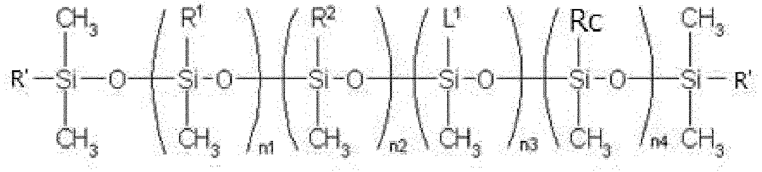 {Formula R¹ is a C6 or lower hydrocarbon group or a hydrogen atom, R² is a C6-30 monovalent hydrocarbon group, L¹ is a specific organosilicon-containing organic group, Rc is a carboxyl group-containing organic group, R' is a group independently selected from the aforementioned R¹, R², L¹ and Rc, but Rc and L¹ are always included in the molecule}
as well as a use thereof.

## Description

### TECHNICAL FIELD

The present invention relates to a carboxylic acid co-modified organopolysiloxane having a specific carboxyl group-containing organic group (hereinafter, referred to as "carboxylic acid-modified group") and a specific silicon-containing organic group in the molecule, which is highly useful as a powder treatment agent, and also relates to applications thereof, and particularly to an aqueous powder dispersion (slurry).

### BACKGROUND ART

Cosmetic compositions containing an aqueous phase as a continuous phase provide a fresh and refreshing feel during use, and therefore have been widely used as make-up cosmetic compositions such as base cosmetic compositions such as emulsions, foundation cosmetic compositions, sunscreen agents, foundations, eye shadows, and the like. In particular, an oil-in-water emulsion cosmetic composition containing an inorganic UV protecting agent typified by hydrophobic fine particulate titanium oxide and hydrophobic fine particulate zinc oxide can be designed to have a high SPF (= Sun Protection Factor) value, which is an index showing the degree of an effect of blocking UV-B waves (wavelength 280 to 315 nm) among ultraviolet rays, and thus has been widely used as a sunscreen cosmetic composition such as sunscreen.

On one hand, oil-in-water emulsified cosmetic materials usually have a problem in that the cosmetic film obtained by applying the cosmetic material has inferior water resistance, and Patent Documents 1 and 2 propose stabilizing the cosmetic material by thoroughly dispersing a hydrophobic powder in the aqueous phase, or by using a carboxylic acid-modified silicone under alkaline conditions to prepare an aqueous slurry that is used as a cosmetic raw material, for the purpose of providing water resistance to the cosmetic film or the like. In particular, Patent Document 2 proposes a carboxylic acid-modified silicone, which may optionally have a siloxane-modified group and a carboxyl group-containing organic group. However, the carboxylic acid-modified silicone specifically proposed in Patent Documents 1 and 2 may not form a stable aqueous slurry for a long period of time when using some hydrophobic powders such as hydrophobic fine particle zinc oxide or the like, leaving room for improvement in powder dispersion properties. On one hand, in Patent Document 3, the applicants proposed the use of a neutral salt of a modified silicone having a carbosiloxane dendrimer structure and a carboxylic acid-modified group in an oil-in-water emulsified cosmetic material, but there is no disclosure of a straight-chain co-modified siloxane molecule that has an organosilicon-containing organic group with a carbosiloxane dendrimer structure or the like on a side chain portion, or of a carboxylic acid co-modified organopolysiloxane having a carboxylic acid-modified group in the molecule. Furthermore, there is also no mention or suggestion of the use of a neutral product of such a carboxylic acid co-modified organopolysiloxane as an aqueous dispersant for a hydrophobic powder. Similarly, Patent Document 4 discloses a powder used in a water-repellent cosmetic material, obtained by treating the powder with a metal cation salt of a carboxylic acid-modified organopolysiloxane, but similarly, there is no disclosure of use of a carboxylic acid co-modified organopolysiloxane containing a specific organosilicon-containing organic group; furthermore, the treated powder is water repellent and is not dispersible in the aqueous phase, and thus the problem of the present invention can not be resolved.

### RELATED ART DOCUMENTS

### PATENT DOCUMENTS

Patent Document 1: International Published Patent WO2015/159773
Patent Document 2: International Published Patent WO2020/036065
Patent Document 3: International Published Patent WO2009/022621
Patent Document 4: Japanese Unexamined Patent Application H9-59125

### SUMMARY OF THE INVENTION

### PROBLEM TO BE SOLVED BY THE INVENTION

In order to resolve the aforementioned problem, an object of the present invention is to provide a new carboxylic acid co-modified organopolysiloxane that can stably and uniformly disperse various hydrophobic powders in an aqueous phase and have excellent function as a powder dispersantor the like. Furthermore, another object is to use this carboxylic acid co-modified organopolysiloxane to provide an aqueous powder composition that has excellent handling workability and that can express functions such as sufficient water repellency, water resistance, sebum resistance, and the like, when blended in a topical agent composition such as a cosmetic material or the like, and to provide a topical agent composition containing this composition.

### MEANS FOR SOLVING THE PROBLEM

As a result of careful examination, the present inventors achieved the present invention by discovering that the above problems can be solved by a straight-chain carboxylic acid co-modified organopolysiloxane that is liquid at 50°C, has carboxyl groups in the molecule, and has one or more organosilicon-containing organic groups (L¹) selected from silylalkyl groups having a carbosiloxy dendron structure and groups having a siloxane macromonomer structure in a specific ratio in a side chain portion.
Furthermore, the present inventors also found that the above problems can be solved by using the co-modified organopolysiloxane as a powder treatment agent, thus arriving at the present invention. Furthermore, the present inventors found that the above problems can be solved by a water-based powder composition using the co-modified organopolysiloxane, a basic substance, a hydrophilic medium, and a powder, such that the powder is uniformly dispersed in an aqueous phase, a topical agent composition (particularly cosmetic material) containing the aforementioned composition, and by a manufacturing method thereof, and thus the present invention was achieved. In particular, the above problems can be resolved very favorably if the carboxylic acid co-modified organopolysiloxane described above has a specific degree of polymerization or the like as described later and is liquid at room temperature (25°C).

### EFFECTS OF THE INVENTION

The present invention can provide a new carboxylic acid co-modified organopolysiloxane that can stably and uniformly disperse various hydrophobic powders in an aqueous phase and have excellent function as a powder dispersantor the like. Furthermore, the present invention can use this carboxylic acid co-modified organopolysiloxane as a powder treatment agent to provide an aqueous powder composition that has excellent handling workability and that can express functions such as sufficient water repellency, water resistance, sebum resistance, and the like, when blended in a topical agent composition such as a cosmetic material or the like, and to provide a topical agent composition containing this composition.

### DESCRIPTION OF THE PREFERRED EMBODIMENT

Hereinafter, the co-modified organopolysiloxane of the present invention, use thereof as various types of treatment agents (surfactant or surface treatment agent), and in particular, use as a powder treatment agent/powder dispersant, and use as a cosmetic raw material will be described below in detail. A detailed description is provided.

The carboxylic acid co-modified organopolysiloxane according to the present invention is a chain polysiloxane that is a liquid at 50°C and is expressed by the structural formula (1) described later, wherein a side chain moiety has an organosilicon-containing organic group (L¹) selected from silylalkyl groups which have a carbosiloxy dendron structure and groups which have a siloxane macromonomer structure, and a specific carboxyl group-containing organic group is provided in the molecule. The carboxyl group-containing organic groups in the carboxylic acid co-modified organopolysiloxane according to the present invention may be introduced on a side chain or at a terminus, but introducing on a chain is particularly preferable. Furthermore, the carboxylic acid co-modified organopolysiloxane according to the present invention need only be liquid at 50°C and 1 atm. For example, it may be solid at room temperature (25°C), but being liquid at room temperature (25°C) is particularly preferable.

The carboxylic acid co-modified organopolysiloxane according to the present invention is expressed by the following structural formula (1)

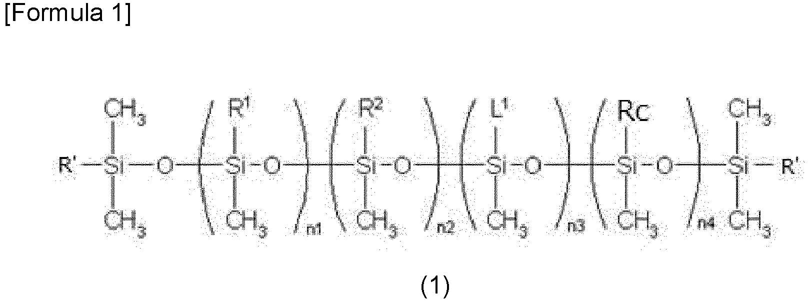

In the formula, R¹ is a substituted or unsubstituted monovalent hydrocarbon group with 1 to 6 carbon atoms or is a hydrogen atom, and does not include other groups corresponding to R², L¹, and Rc, but a methyl or phenyl group is industrially preferable.

R² is a substituted or unsubstituted, straight-chain or branched monovalent hydrocarbon group with 6 to 30 carbon atoms, may be optionally included in the molecule, and may improve affinity with non-silicone oil agents such as hydrocarbon-based oil agents (cosmetic raw materials). Suitably, R² is an alkyl group with 6 to 30 carbon atoms, and examples include hexyl groups, heptyl groups, octyl groups, decyl groups, dodecyl groups, undecyl groups, and hexadecyl groups, but alkyl groups with 8 to 20 carbon atoms are particularly preferred.

L¹ is a silicon-containing organic group selected from silylalkyl groups having a carbosiloxy dendron structure and groups having a siloxane macromonomer structure. A certain ratio of the specific carboxyl group-containing organic group which are Rc and L', at least one of which is present in a side chain moiety, in the molecule of the carboxylic acid co-modified organopolysiloxane according to the present invention provides excellent powder dispersion performance, particularly in an aqueous phase. More specifically, if i = 1, L¹ in the present invention is one or more type selected from:
a silylalkyl group having a siloxane dendron structure, expressed by the following general formula (2): 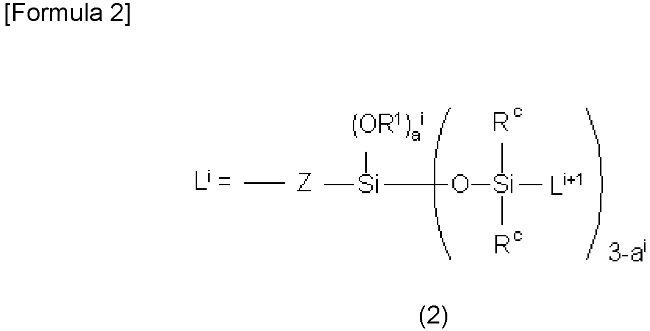
(where R¹ is a group similar to the groups described above, R^{C} is an alkyl group with 1 to 6 carbon atoms, or a phenyl group, and Z is a bivalent organic group.
i indicates the number of units of silylalkyl groups represented by Lⁱ and is an integer from 1 to c where c represents the number of units, or in other words the number of repetitions of the silylalkyl group, and the number of units c is an integer from 1 to 10. Lⁱ⁺¹ represents a silylalkyl group when i is less than c, and is a methyl group or a phenyl group when i=c.
aⁱ is a number from 0 to 3);
a chain organosiloxane group, expressed by the following general formula (2'): 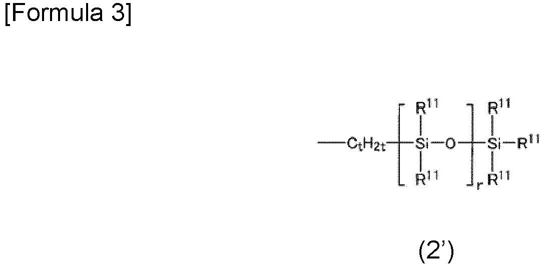
(where R¹¹ are each independently a substituted or unsubstituted monovalent hydrocarbon group having 1 to 30 carbon atoms, hydroxyl groups, or hydrogen atoms, and at least one of the R¹¹ groups is a monovalent hydrocarbon group.
t is a number in a range of 2 to 10; and r is a number in a range of 1 to 500); and
a chain type organosiloxane group expressed by the following general formula (2"): 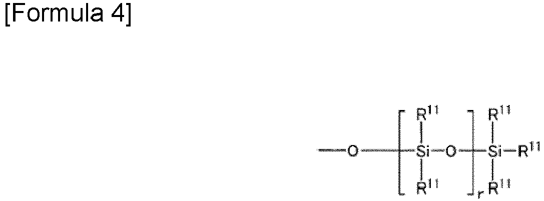 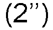
(where R¹¹ and r are similar to those described above).

The groups expressed by L¹ may be the same or different. For example, a silylalkyl group having a carbosiloxy dendron structure and a group having a siloxane macromonomer structure may be present in the same molecule. The silylalkyl group may be a combination of organosilicon-containing organic groups with different degrees of branching (generations), and the group having a siloxane macromonomer structure may be a combination of organosilicon-containing organic groups with different degrees of polymerization.

The group expressed by L¹ may be a silylalkyl group having a carbosiloxane dendrimer structure and is defined as the silylalkyl group expressed by the above general formula (2) when i = 1. The silylalkyl group having the carbosiloxane dendrimer structure has a structure in which the carbosiloxane units are extended in the form of a dendrimer. Therefore, the silylalkyl group is a functional group exhibiting increased water repellency in comparison to straight-chain or simply branched polysiloxane units. Furthermore, the carboxylic acid co-modified organopolysiloxane according to the present application can provide a high degree of powder dispersibility, without impeding the feel derived from the hydrophilic functional group. Furthermore, the silylalkyl group having a carbosiloxane dendrimer structure is chemically stable; therefore, the silylalkyl group is a functional group providing advantageous properties that can be used in combination with a wide range of cosmetic material components.

In general formula (2), R^{C} is an alkyl group with 1 to 6 carbon atoms or a phenyl group, but methyl or phenyl groups are industrially preferable.

In general formula (2), i represents the number of units of silylalkyl groups expressed by Lⁱ and is an integer from 1 to c, where c represents the number of units, or in other words the number of repetitions of silylalkyl groups, and where the number of units c is an integer from 1 to 10. Lⁱ⁺¹ represents a silylalkyl group when i is less than c, and is a methyl group or a phenyl group when i=c. In particular, it is preferably a methyl group when i = c.

Note that the number of units c is preferably an integer from 1 to 3 and is more preferably 1 or 2 from an industrial standpoint.
For each of the number of units, the group expressed by L¹ is expressed as follows. In the formula, R² and Z are the same groups as described above.

If the number of units c = 1, L¹ is expressed by the following general formula (2-1).

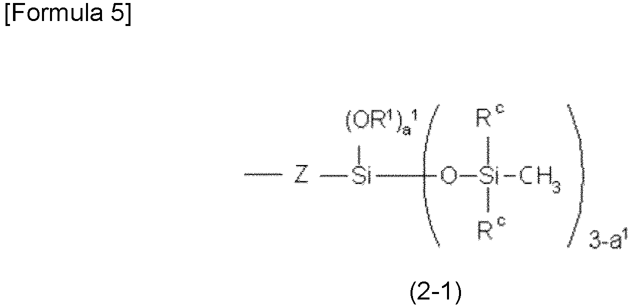

When the number of units is c = 2, L¹ is expressed by the following general formula (2-2).

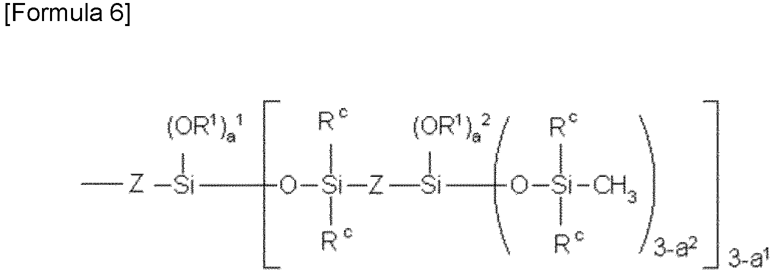

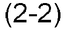

In formula (2), ai independently represents a number in the range of 0 to 3. In the structures expressed in formulas (2-1) to (2-3) where the number of units is 1 to 3, a1, a2 and a3 independently represent a number in the range of 0 to 3. ai values are preferably numbers within the range of 0 to 1, and ai is particularly preferably 0.

In the formula, Z are each independently a bivalent organic group, and specific examples thereof include a bivalent organic group formed by addition-reacting a silicon-bonded hydrogen atom and a functional group having an unsaturated hydrocarbon group such as an alkenyl group, an acryloxy group, a methacryloxy group, or the like at the terminal. Depending on the method for introducing the silylalkyl group having a carbosiloxane dendrimer structure, the functional group can be appropriately selected and is not restricted to the functional groups described above. Z in the present invention is preferably an alkylene group with 2 to 10 carbon atoms, but ethylene groups are most preferable.

The group expressed by L¹ may be a siloxane macromonomer structure-containing group bonded to silicon atoms constituting the main chain via an oxygen atom or an alkylene group, and is a functional group that is hydrophobic and exhibits certain water-repellent/lipophilic properties, without impairing the feel derived from hydrophilic functional groups, and provides a high degree of powder dispersibility to the carboxylic acid co-modified organopolysiloxane according to the present application. Specifically, the group expressed by L¹ may be a functional group expressed by the general formula (2') and general formula (2"). In the formula, R¹¹ is particularly industrially preferably a methyl group, phenyl group or hydroxyl group, and at least a portion of R¹¹ is a methyl group, and a portion is preferably in the form of a long chain alkyl group with 8 to 30 carbon atoms. However, at least one R¹¹ is a monovalent hydrocarbon group such as a methyl group or the like, t is a number in the range of 2 to 10, and r is a number in the range of 1 to 500. From the viewpoint of compatibility with various oil agents, r is preferably a number in the range of 1 to 100, and r is particularly preferably a number in the range of 2 to 30.

Rc represents a carboxyl group-containing organic group expressed by the general formula: -Rc¹-(ORc²)p-(O)w-Rc³-COOH, (where Rc¹ represents a straight-chain or branched alkylene group having 2 to 22 carbon atoms, Rc² represents a straight-chain or branched alkylene group having 2 to 4 carbon atoms, Rc³ represents a bond (-) or a straight-chain or branched alkylene group having 1 to 22 carbon atoms, p represents a number from 0 to 200, and w represents a number of 0 or 1). A salt neutralized by the molecular coexistance of a specific silicon-containing organic group which is L', and particularly by a basic substance, can provide excellent powder dispersibility in the aqueous phase.

In the general formula expressing the aforementioned carboxyl group-containing organic group, Rc¹ is a straight-chain or branched alkylene group with 2 to 22 carbon atoms, preferably 2 to 12 carbon atoms, and particularly preferably 2 to 10 carbon atoms, such as a decamethylene group, or the like.

In addition, examples of the straight-chain or branched alkylene group having 2 to 4 carbon atoms of Rc² include ethylene, propylene, trimethylene and butylene groups.

Rc³ may be a bond (-), and may be a straight-chain or branched alkylene group with 1 to 22 carbon atoms.

p represents the number of 0 to 200, and the number of 0 to 20 is preferable, and the number of 0 to 10 is particularly preferable. In addition, w represents the number of 0 or 1, and is preferably 0. Note that, when p and w are both 0, if Rc³ is a bond, the carboxyl group-containing organic group is expressed by the structural formula -(CₙH₂ₙ)-COOH, and the carboxyl group-containing organic group preferably has a structure in which one carboxyl group is bonded to a silicon atom via a straight-chain or branched alkylene group. Note that in the formula, n is preferably a number from 3 to 20, and particularly preferably a number from 3 to 16.

R' is a group independently selected from R¹, R², L¹ and Rc described above, and if R' is L', the structure has an additional silicon-containing organic group at the end of the molecular chain, and if R' is Rc, the structure has a carboxyl group-containing organic group at the end of the molecular chain, and is preferably a methyl group. However, when n4 is 0, as described later, at least one R' is Rc, since the carboxylic acid co-modified organopolysiloxane according to the present invention has one or more Rc.

In structural formula (1), n1 to n4 represent the average degree of polymerization of the diorganosiloxy unit, and (n1 + n2 + n3 + n4) is a number in the range of 1 to 1000, preferably 2 to 50, and particularly preferably 2 to 20. In other words, the carboxylic acid co-modified organopolysiloxane according to the present invention preferably has relatively low polymerization, particularly from the perspective of powder dispersion performance in the aqueous phase. In particular, if the degree of polymerization is relatively low, the composition will be a liquid with low viscosity at room temperature (25°C), and this is also advantageous in creating a water-based slurry.

Furthermore, in structural formula (1), n1 is a number in the range of 0 to 999, more preferably 0 to 49, and most preferably 0 to 5. n2 is a number in a range of 0 to 100, more preferably from 0 to 10. n3 is a number in a range of 1 to 100, more preferably from 1 to 10. n 4 is a number in a range of 0 to 100, more preferably from 0 to 10.

From the perspective of technical effect according to the present invention, n1 to n4 are numbers in the range where the carboxylic acid co-modified organopolysiloxane of the present invention becomes liquid at room temperature (25°C), and having low viscosity is particularly preferable. The carboxylic acid co-modified organopolysiloxane that is in a liquid form at room temperature (25°C) and has low viscosity has advantages in that dispersion effects are excellent for hydrophobic powders and no heating operation is required when forming the aqueous dispersion composition.

From the perspective of the technical effect according to the present invention, it is particularly preferable if in the above structural formula (1) of the carboxylic acid co-modified organopolysiloxane of the present invention, (n1 + n2 + n3 + n4) is a number in a range of 2 to 20, n3 is a number in a range of 1 to 10, n4 is a number in a range of 1 to 10, and (n1 + n2 + n3)/n4 is in a range of 0.1 to 5.0. The carboxylic acid co-modified organopolysiloxane having such a structure will have a structure in which a certain ratio of silicon-containing organic groups (L¹) and carboxyl group-containing organic groups (Rc) are present on side chains of the molecular chain, and thus will have excellent powder treatment effects and further improved uniform dispersibility of hydrophobic powders in the aqueous phase, and thus when an aqueous dispersion composition containing the carboxylic acid co-modified organopolysiloxane is used as a cosmetic raw material, it has the advantage of effectively achieving functions of sufficient water repellency, water resistance, sebum resistance, and the like. There is also an advantage in that the uniform dispersion state of the hydrophobic powder in the aqueous phase is maintained for a long period of time.

The carboxylic acid co-modified organopolysiloxane according to the present invention can be manufactured by a method to obtain the target substance by reacting a compound having an organosilicon-containing organic group having one carbon-carbon double bond at one end of the molecular chain (for example, the silylalkyl group) with an organopolysiloxane having a reactive functional group such as Si-H or the like, and then performing an addition reaction to add an unsaturated carboxylic acid ester compound, an unsaturated carboxylic acid silyl ester, or an allyloxycarboxylic ester, in the presence of a platinum catalyst, and then performing saponification or hydrolysis after the reaction.

### [Use of the carboxylic acid co-modified organopolysiloxane]

The novel carboxylic acid co-modified organopolysiloxane according to the present invention has a hydrophilicity carboxyl group-containing organic group and a silicon-containing organic group having high water repellency in a single molecule, and preferably has low viscosity. Therefore, handling, working efficiency, and compounding stability with various types of cosmetic raw materials are superior, and therefore useful as various types of treatment agents and cosmetic raw material components. In particular, this compound is extremely useful as a powder surface treatment agent, surfactant, or powder dispersant, for use in surface treating a hydrophobic powder used in cosmetic material or dispersing a powder in an aqueous phase.

### [Use as a powder treatment agent or powder dispersant]

The carboxylic acid co-modified organopolysiloxane according to the present invention functions as a surface treatment agent or dispersant for powders, particularly hydrophobic powders, and can be used for hydrophobic powders, suitably in combination with a basic substance, to form an aqueous powder composition with uniform and stable dispersion of a hydrophobic powder in the aqueous phase. In particular, a carboxylic acid co-modified organopolysiloxane composition containing (A) the carboxylic acid co-modified organopolysiloxane according to the present invention, (B) a basic compound, and (C) one or more hydrophilic medium selected from water, polyhydric alcohols, and lower alcohols, forms a carboxylate structure with a partially neutralized carboxylic acid residual group, and therefore is particularly useful as a powder surface treatment agent, surfactant or surface treatment agent, which are dispersants for hydrophobic powders.

### Powder composition

The present invention relates to a powder composition, containing:
(A) the carboxylic acid co-modified organopolysiloxane according to the present invention;
(B) a basic compound;
(C) one or more hydrophilic medium selected from water, polyhydric alcohols and lower alcohols; and
(D) a powder or colorant. The powder composition preferably contains (D1) a hydrophobic powder or colorant, and has a structure in which component (D1) is dispersed in the aqueous phase. The powder composition containing the carboxylic acid co-modified organopolysiloxane according to the present invention can be suitably used as topical agent composition, and particularly a cosmetic material or a cosmetic raw material.

The powder composition according to the present invention is an aqueous dispersion composition with a continuous layer of an aqueous phase suitably containing a hydrophilic medium, and is a composition in which a powder or colorant, particularly a hydrophobic powder or colorant, are dispersed in a hydrophilic medium, and is sometimes called a "water-based slurry" because these compositions generally have fluidity. Note that the hydrophilic medium of the present invention refers to an aqueous dispersion medium containing water, one or more alcohols selected from polyhydric alcohols, lower alcohols such as ethyl alcohol, and the like, that are uniformly miscible with water, and salts, such as basic substances, and the like, and is particularly preferably substantially free of an oil agent/oil-based cosmetic raw material capable of forming an independent oil phase without being miscible with water such as an oil agent.

Note that the carboxylic acid co-modified organopolysiloxane according to the present invention can be similarly used as the carboxylic acid-modified silicone specifically disclosed in the aforementioned Patent Documents 1 and 2, and is useful for easily preparing a water-based slurry with low viscosity and excellent long-term storage stability even for inorganic powders such as zinc oxide or the like, that cannot be sufficiently processed with these previously known carboxylic acid-modified silicones.

### [(B) Basic substance]

The powder composition of the present invention preferably contains at least one (B) basic substance. The component (B) is capable of anionizing the carboxylic acid-modified moiety of (A) the carboxylic acid co-modified organopolysiloxane according to the present invention, and improving the function of the surfactant or powder dispersant. In particular, the aqueous dispersion composition of the present invention contains (D) a powder or colorant, preferably (D1) a hydrophobic powder or colorant; however, by using the component (A) and the component (B) in combination, it is possible to favorably disperse the component (D) in the aqueous phase as compared to using the component (A) alone so that it contributes to not only improving the uniform dispersibility and stability of the aqueous dispersion composition body, but also achieving sufficient water repellency, water resistance, and sebum resistance of the cosmetic material compounded with the aqueous dispersion composition as a cosmetic raw material.

The basic substance used in the present invention is not particularly limited as long as the basic substance is a compound that exhibits basicity when dissolved in water, and various types of inorganic compounds and organic compounds can be used. One or more types of the basic substances may be compounded.

Examples of the organic compounds include monoethanolamine, triethanolamine, 2-amino-2-methyl-1,3-propanediol, aminomethylpropanol, aminomethyl propanol, 2-amino-2-hydroxymethyl-1,3-propanediol, arginine, guanidine, and the like.

Examples of the inorganic compounds include sodium hydroxide, sodium carbonate, potassium hydroxide, potassium carbonate, calcium hydroxide, calcium carbonate, ammonia, and the like. Among these, potassium hydroxide can be particularly suitably used.

The compounding amount of the basic compound in the aqueous dispersion composition of the present invention is not particularly limited, and when the base is a monovalent base per 1 mol of the carboxylic acid groups contained in the compounded (A) carboxylic acid co-modified organopolysiloxane, the carboxylic acid group/monovalent base (molar ratio) is preferably 1/0.5 to 1/1.5.

The pH of the aqueous dispersion composition of the present invention may be acidic or alkaline, and from the perspective of anionizing the carboxylic acid-modified moiety of the aforementioned (A) carboxylic acid co-modified organopolysiloxane and improving the dispersibility of the powder or colorant as component (D), is preferably weakly acidic to alkaline, and specifically, the pH of the total composition is preferably in the range of 6.5 to 14.0, may be in the range of pH 7.1 to 10.0, and is more preferably in the range of pH 7.2 to 9.5.

### [(C) Hydrophilic medium]

The hydrophilic medium is the primary dispersion medium of the powder composition of the present invention, and is one or more medium selected from water, polyhydric alcohols, and lower alcohols, which form the aqueous phase of the composition. The powder composition of the present invention is suitably an aqueous dispersion, and in the presence of the aforementioned component (A) and component (B), component (D), a powder or colorant, preferably forms a stable dispersed structure in the aqueous phase.

The hydrophilic medium of the present invention preferably contains water. On the other hand, (A) carboxylic acid co-modified organopolysiloxane has a silicon-containing organic group and a carboxyl group-containing organic group that exhibit high water repellency in the same molecule. Therefore, unlike conventionally known carboxylic acid-modified silicones, this composition may form an aqueous dispersion composition with excellent uniform dispersibility and stability in the aqueous phase of component (D), which is a powder or colorant, even without containing one type selected from polyhydric alcohols and lower alcohols (particularly ethyl alcohol).

When the hydrophilic medium in the present invention contains polyhydric alcohol and lower alcohol (in particular, ethyl alcohol), it likewise has excellent uniform dispersibility and stability in the aqueous phase of the powder or colorant that is component (D), and when the aqueous dispersion composition of the present invention is used as a cosmetic raw material, a sense of moisturizing and usability may be achieved. In particular, when used as a raw material in cosmetic materials or the like, the hydrophilic medium that is component (C) in the powder composition preferably contains water, but component (C) may be a mixture of one or more selected from the following: (C1) water and (C2) a polyhydric alcohol and lower alcohol (suitably, ethanol).

Examples of the polyhydric alcohols include sorbitol, xylitol, propylene glycol, dipropylene glycol, 1,3-butylene glycol, glycerin, diglycerin, polyethylene glycol, and the like, and these polyhydric alcohols can be used alone or in combination of two or more types. On the other hand, methyl alcohol and ethyl alcohol are representative of lower alcohols, but from the perspective of use as a cosmetic raw material, non-toxic ethyl alcohol is particularly preferred. Lower alcohols can be used alone or in combination with the polyhydric alcohols mentioned above. Specifically, the ethyl alcohol and the polyhydric alcohol can be mixed at any mass ratio of 100:0 to 0:100, or used alone in the present invention, but in this case, water may or may not be added.

### [(D) Powder or colorant]

The powder or colorant (D) used in the powder composition or the like according to the present invention (particularly an aqueous slurry) is a component that is commonly used in a cosmetic material and includes white and colored pigments as well as extender pigments. The white and colored pigments are used for coloring cosmetic compositions and the like, while extender pigments are used for improving the tactile sensation of the cosmetic compositions. As the "powder" of the present invention, white and colored pigments commonly used in cosmetic compositions, as well as extender pigments can be used without particular limitation. In the present invention, preferably, one or two or more of the powders are compounded. The form (sphere, bar, needle, plate, amorphous, spindle, cocoon, or the like), particle size (aerosol, fine particle, pigment-grade particle, or the like), and particle structure (porous, nonporous, or the like) of the powder are not limited in any way, but an average primary particle size is preferably in a range of 1 nm to 100 µm. Particularly, when compounding the powder or coloring agent as a pigment, preferably one or two or more selected from an inorganic pigment powder, an organic pigment powder, and a resin powder having an average particle size in a range of 1 nm to 20 µm is compounded.

Examples of the powder include inorganic powders, organic powders, surfactant metal salt powders (metallic soaps), colored pigments, pearl pigments, metal powder pigments, and the like. Compounded products of these pigments can be used. Furthermore, the surface of the pigment may be treated to be hydrophobic or water repellent. In particular, with the present invention, a suitable component (D) is a hydrophobically treated, (D1) hydrophobic powder or colorant. Specific examples of hydrophobic treatments and hydrophobic powders are common to the hydrophobic powders or colorants disclosed by the applicants in paragraphs 0043 to 0054 of Patent Document 2 (International Published Patent WO2020/036065).

Hydrophobic powders are difficult to disperse in the aqueous phase because their surfaces are hydrophobic, but by using the carboxylic acid co-modified organopolysiloxane of the present invention, compositions in which a large amount of hydrophobic powder or colorant is uniformly and stably dispersed in the aqueous phase (including use as a cosmetic raw material or topical agent composition) can be achieved. The carboxylic acid co-modified organopolysiloxane has the advantage that a low-viscosity aqueous slurry can be prepared even for inorganic powders such as zinc oxide, for which sufficient dispersibility cannot be achieved under the same conditions as with a conventional carboxylic acid-modified silicone.

Preferably, the hydrophobic powder used in the present invention is an inorganic powder having an UV light absorbing/scattering ability such as titanium oxide, zinc oxide, iron oxide, cerium oxide, and composites thereof, whose surface has been subjected to a hydrophobizing treatment. The composite is preferably a surface-treated powder, a powder in which other inorganic particles are dispersed in the inorganic powder, or the like. Particularly suitable are (D1-1) hydrophobized fine particle titanium dioxide and hydrophobized fine particle zinc oxide having an average particle size in the range of 1 to 200 nm, and by using the carboxylic acid co-modified organopolysiloxane of the present invention together with components (B) and (C), the powder composition, in particular an aqueous dispersion composition, is formed with the powder or colorant uniformly and stably dispersed in the aqueous phase.

### [Powder composition as raw material for cosmetic materials and the like, and composition thereof]

The powder composition containing a carboxylic acid co-modified organopolysiloxane of the present invention and a powder or colorant is suitably an aqueous dispersion composition having an aqueous phase containing a hydrophilic medium as a continuous layer, is a composition in which a powder or colorant, in particular a hydrophobic powder or colorant, is dispersed in a hydrophilic medium, and is used as a raw material for use in compositions of cosmetic materials, paints, inks, and the like (hereinafter, referred to as "cosmetic materials, and the like").

When used as a raw material for a cosmetic material or the like, the powder composition preferably contains 80 mass% or more of the above components (A) through (D) relative to the total composition, and 90 mass% or more is more preferred. Furthermore, the powder composition according to the present invention can form an aqueous slurry with low viscosity, even though containing a relatively large amount of a powder or colorant; therefore, it is possible to include component (D), a powder or colorant, preferably (D1) a hydrophobic powder or colorant, in the range of 10 to 70 mass% of the total composition, suitably in the range of 15 to 65 mass%, more preferably in the range of 15 to 60 mass%, and thus a composition in which the powder or colorant is uniformly and stably dispersed in the aqueous phase can be achieved.

When used as a raw material in a cosmetic material or the like, component (A) in the powder composition preferably contains 5 to 30 parts by mass relative to 100 parts by mass of component (D), particularly component (D1) hydrophobic powder or colorant, but 10 to 25 parts by mass is more preferred. If the compounding amount of the component (A) is less than the lower limit described above, dispersion of a hydrophobic powder or colorant, in particular, in the aqueous phase may be insufficient, and if the compounding amount of the component (A) exceeds the upper limit described above, it may not be possible to compound the component (D) in the powder composition which is an aqueous slurry in a sufficient amount.

When used as a raw material in a cosmetic material or the like, the hydrophilic medium that is component (C) in the powder composition preferably contains water. Specifically, if component (C) contains one or more selected from the following: (C1) water and (C2) a polyhydric alcohol and lower alcohol (suitably, ethanol), the content of component (C1), based on the total mass of the powder composition, is preferably in the range of 10 to 80 mass%, more preferably 15 to 70 mass%, and particularly preferably 20 to 65 mass%. Furthermore, component (C2) is preferably 0.1 to 50 mass%, more preferably 0.5 to 45 mass%, even more preferably 1 to 40 mass%, and particularly preferably 2 to 35 mass%, based on the total mass of the powder composition.

When used as a raw material for a cosmetic material or the like, the powder composition of the present invention preferably does not contain a component other than the aforementioned components in a proportion exceeding 20 mass%, more preferably 10 mass% or less, as long as the performance of the dispersion is not impaired. However, it is preferable not to add a compound or the like that may impair the stability of the dispersion. In particular, the addition of the oil agent is not preferable.

Optionally, the component that may be added is not particularly limited, and examples thereof include preservatives. That is, the aqueous dispersion composition formed of the aforementioned components may cause spoilage depending on the storage conditions and the storage period. The preservatives may be added to prevent such spoilage. Such preservatives are not particularly limited, and examples thereof include glycol-based preservatives such as propylene glycol, butylene glycol, dipropylene glycol, pentylene glycol, hexylene glycol, and the like, parabenzoic acid esters such as methyl paraoxybenzoate and the like, piroctone olamine, phenoxyethanol, caprylsanpolyglyceryl-3, and the like. These can be added for each component within a range that exhibits antiseptic performance, and that does not adversely affect the performance of the carboxylic acid co-modified organopolysiloxane which is a dispersant. Among these, it is particularly preferable to use pentylene glycol and 1,3-butylene glycol. From the perspective of applicability, it is preferable that the components other than carboxylic acid co-modified organopolysiloxane according to the present invention, powder or colorant, water, and preservative be 1% or less. Herein, the glycols are often used in cosmetics as moisturizers, but they also have antibacterial properties, and therefore have an effect as preservatives.

These components are components that overlap with a part of the above component (C), by using polyhydric alcohol as the component (C), the storage stability and moisturizing function of the powder composition, particularly the water-based slurry of the present invention, may be improved without adding another preservative, moisturizer, or the like.

The dispersion method for obtaining the powder composition of the present invention, especially a composition in which the powder or colorant is dispersed in a hydrophilic medium, is not restricted in particular, as long as it is a known method that can uniformly disperse each component using mechanical force, and a method using a bead mill, a method of using a highpressure homogenizer, and a method using a triple roller, and the like, are preferable.

The powder composition obtained by the present invention can be applied to prepare a thin film. For example, a zinc oxide film that is fixed with silica can be produced by applying a silicone-treated zinc oxide dispersion and heat treating with high heat, plasma, or the like. Further, when a dispersant such as a polyether or a polyether alkyl ether is used as a dispersant for a similar zinc oxide dispersion, a silicone-coated zinc oxide coating film can be easily produced by heating at a temperature lower than the decomposition temperature of the silicone coated with zinc oxide. Note that it goes without saying that other powders can also be used for forming a coating film.

### [Use of aqueous dispersion composition: Material for cosmetic composition or the like]

In addition, the aqueous dispersion composition of the present invention can be compounded as is into the cosmetic compositions, paints, and inks. In this case, a cosmetic composition, an aqueous paint, and an ink composition can be obtained by mixing the various components used in the dispersion, cosmetic composition, paint, ink, and the like. In particular, the aqueous dispersion composition of the present invention is suitable as a material for a cosmetic composition that is compounded directly into a cosmetic composition.

The cosmetic composition obtained in this manner can suitably disperse the hydrophobic powder in the aqueous phase of the water-based composition or emulsion. As a result, a function such as sufficient water repellency, water resistance, sebum resistance, and the like can be obtained by a cosmetic composition or the like in which the water-based dispersion is compounded.

The cosmetic composition is not particularly limited, and by mixing the material for a cosmetic composition as necessary into the aqueous dispersion composition of the present invention, a base make-up cosmetic composition such as a sunscreen agent or the like; a base makeup cosmetic composition such as foundations; a point makeup cosmetic composition such as lipstick; and the like. That is, the aqueous dispersion composition of the present invention can be used directly in the production of cosmetic compositions.

The cosmetic composition can be in any form of an oil-based cosmetic composition, an aqueous cosmetic composition, an O/W cosmetic composition, or a W/O type cosmetic composition. In particular, the compositions are particularly suitable for use in sunscreen cosmetic materials (including sunscreen agents) because they can contain large and stable amounts of an inorganic UV light blocking agent.

The cosmetic composition may be a combination of any aqueous component or oily component that can be used in the cosmetic field. The aqueous component and the oily component are not particularly limited, and examples thereof include oil agents, surfactants, moisturizers, higher alcohols, metal ion sequestering agents, natural and synthetic polymers, water-soluble and oil-soluble polymers, UV blocking agents, various extracts, coloring agents such as organic dyes, preservatives, antioxidants, pigments, thickeners, pH adjusters, fragrances, cooling agents, antiperspirants, bactericides, skin activators, and those containing components such as various powders.

These components are, for example, available without limitation as specifically disclosed in Patent Document 1 and Patent Document 2.

When the aqueous dispersion composition of the present invention is compounded into a paint, a resin in the paint may be curable or non-curable. The paint may be a solvent-based paint containing an organic solvent or a water-based paint in which a resin is dissolved or dispersed in water.

When the aqueous dispersion composition of the present invention is used as an added component to a paint composition, it can be used in combination with a coating film forming resin such as an acrylic resin, a polyester resin, and an epoxy resin; various pigments such as a coloring pigment, an extender pigment, and a bright pigment; and a curing catalyst, a surface conditioner, a defoamer, a pigment dispersant, a plasticizer, a film-forming aid, a UV absorber, and an antioxidant.

Also, the aqueous paint and aqueous ink composition thus obtained is preferable in that the hydrophobic powder is stably dispersed in the aqueous medium.

### [Topical agent compositions]

As described above, the carboxylic acid co-modified organopolysiloxane according to the present invention and compositions or the like containing the same can be used as cosmetic raw materials in topical agent compositions. These topical agent compositions may include the carboxylic acid co-modified organopolysiloxane (=component (A)), may include a carboxylic acid co-modified organopolysiloxane composition containing the components (A), (B), and (C), and having a structure in which the carboxylic acid-modified groups have been partially neutralized in a hydrophilic medium, and may include a carboxylic acid co-modified organopolysiloxane composition containing the above components (A), (B), (C), and (D) and having a structure in which component (D), which is a powder or colorant, is dispersed in a hydrophilic medium.

A topical agent composition according to the present invention particularly suitably contains components (A), (B), (C), and (D) and preferably has a structure in which component (D), which is a powder or colorant, is dispersed in the aqueous phase. As described above, by using the carboxylic acid co-modified organopolysiloxane according to the present invention, (D1) hydrophobic powders or colorants can be stably dispersed in the aqueous phase, in particular, (D1-1) a hydrophobic fine particle titanium dioxide or hydrophobic fine particle zinc oxide, with an average particle size in the range of 1 to 200 nm, can be stably and uniformly dispersed in large amounts in the aqueous phase, and is especially useful as an inorganic UV light blocking agent. Therefore, the storage stability of the topical agent composition and the UV light blocking performance is high, and a topical agent composition with excellent water resistance of the film (cosmetic film) can be provided, while preventing loss of feeling to touch and sensation during use, as much as possible. In particular, it is anticipated that the carboxylic acid co-modified organopolysiloxane according to the present invention can be used to provide a sunscreen cosmetic material or the like that provides both an increased degree of freedom in formulation design, and performance such as water resistance and the like, sensation during use, and UV light blocking properties represented by the SPF value.

The external use preparation according to the present invention is not particularly limited, provided that it is a composition for application to the human body as a cosmetic composition or a medicament. Specific examples of cosmetic composition products of the present invention include skin cleansing agent products, skin care products, makeup products, antiperspirant products, ultraviolet light blocking products, and similar skin use cosmetic products; hair use cleansing agent products, hair dressing products, hair use coloration products, hair growth products, hair rinsing products, hair conditioning products, hair treatment products, and similar hair use cosmetic products; and bath use cosmetic products. Examples of the medicament of the present invention include hair regrowth agents, hair growth promoters, analgesics, germicides, anti-inflammatory agents, refreshing agents, and skin anti-aging agents, but are not limited thereto.

The topical agent composition according to the present invention may and preferably does contain one or more additive selected from oil agents, carboxylic acid-modified silicones other than component (A), salts of higher fatty acids, various surfactants (one or more selected from anionic, cationic, nonionic, amphoteric, and which may be cleaning agent components), water-soluble thickeners, oil-soluble film-forming agents, water-soluble UV light absorbers, and vinyl polymer emulsions. Note that specific examples of these components can be the same as those proposed by the applicant in International Patent Application (PCT/JP19/029957) as well as in Patent Document 1 (WO2015/159773) mentioned above. The combined use of these components (for example, vinyl polymer emulsions) may further improve the dispersibility of powders and other substances in the aqueous phase and achieve the effect of increasing the SPF value of the overall cosmetic material.

Other components ordinarily used in cosmetic materials can be added to the topical agent composition according to the present invention within a range that does not hinder the effect of the present invention, examples including: hydrophilic powders, moisturizers other than component (C), gelling agents, preservatives, antimicrobial agents, fragrances, salts, antioxidants, pH adjusters other than component (B), chelating agents, refreshing agents, anti-inflammatory agents, physiologically active components (skin lightening agents, cell activating agents, rough skin improving agents, circulation promoters, skin astringents, anti-seborrheic agents, etc.), vitamins, amino acids, nucleic acids, hormones, inclusion compounds, and the like. Other components are not particularly limited.

### [Common uses and replacements for conventional carboxylic acid-modified silicones, particularly commercial products]

The carboxylic acid co-modified organopolysiloxane according to the present invention can be used in common with carboxylic acid-modified silicones in cosmetic materials and other applications proposed by the applicants in the above-mentioned Patent Document 1 (International Published Patent WO2015/159773) and Patent Document 2 (International Published Patent WO2020/036065), and can be used to replace some or all of the carboxylic acid-modified silicone. Similarly, the carboxylic acid co-modified organopolysiloxane according to the present invention can be used in applications common with carboxylic acid modified silicones in the formulation, such as in cosmetic materials and the like proposed by the applicant in International Patent Applications (PCT/JP19/029957, PCT/JP19/029927), International Published Patent WO/2020/036064, International Published Patent WO/2020/036061, International Published Patent WO/2020/036062, and International Published Patent WO/2020/036063, and the carboxylic acid co-modified silicone in the formulation can partially or completely replace the carboxylic acid-modified silicone. It may be possible to further improve the uniform dispersibility, storage stability, and the like of hydrophobic powders or colorants in the formulation without compromising the advantageous technical effect, feel and texture of the cosmetic material or the like containing these carboxylic acid modified silicones.

Furthermore, the carboxylic acid co-modified organopolysiloxane according to the present invention can be used in applications in common with carboxylic acid-modified silicone products that the applicant in this case markets under the product name ES-5800 Formulation Aid (manufactured by Dow Toray Industries, Inc.) and the like. The applicant hereby expressly teaches the use of such carboxylic acid modified silicone products in the methods and formulations of cosmetic materials and the like disclosed for such carboxylic acid-modified silicone products, replacing some or all of the carboxylic acid-modified silicone products.

### Embodiments

Hereinafter, the present invention will be described in more detail based on Embodiments, but the present invention is not limited to these Embodiments. The compounding amount of each component is "% by mass" ("wt%") unless otherwise specified. Furthermore, in the structural formulas, Me represents a methyl group and Bu represents a butyl group.

### [Examples 1 to 4]

The carboxylic acid co-modified organopolysiloxanes (compounds 1-4) according to the present invention were synthesized by the following method.

### [Example 1]

In a flask equipped with a stirrer, a reflux condenser, a dropping funnel, and a thermometer, 126.90 g of trimethylsilyl undecylenate, 78.90 g of tristrimethylsiloxyvinylsilane, and 0.025 g of platinum-1,3-divinyl-1,1,3,3-tetramethyldisiloxane complex were added, and then 94.20 g of methylhydrogenpolysiloxane expressed by the following general formula was added dropwise while maintaining a temperature range of 70°C to 80°C.

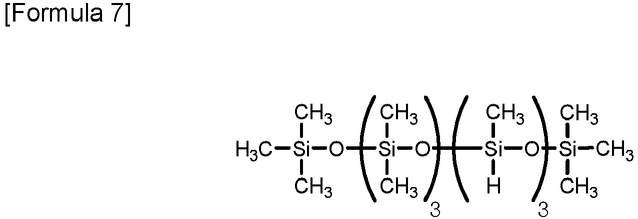

After dropping was complete, the mixture was cured for 3 hours at 110°C, and then the completion of the reaction was confirmed by a hydrogen generation method. The low boiling point content was distilled off under reduced pressure. Thereafter, 45 g of ion exchanged water was added, curing was performed with reflux for 4 hours, and deprotection was performed. Thereafter, the low boiling point content was again removed under reduced pressure to obtain a compound 1. As a result of the analysis, it was confirmed that it was the compound 1 represented by the following chemical structural formula.

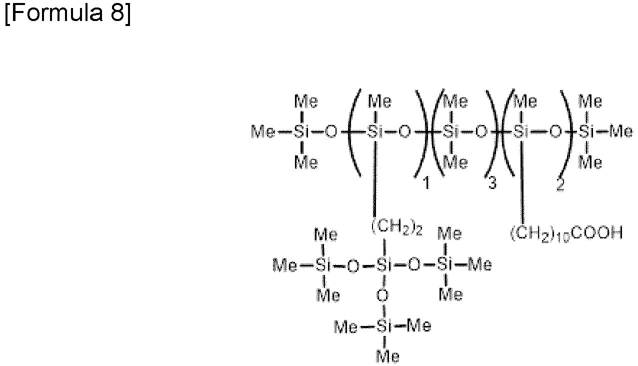

### [Example 2]

In a flask equipped with a stirrer, a reflux condenser, a dropping funnel, and a thermometer, 44.54 g of trimethylsilyl undecylenate, 52.29 g of a macromonomer raw material expressed by Bu-(OSiMe₂)₆-CH=CH₂, and 0.018 g of platinum-1,3-divinyl-1,1,3,3-tetramethyldisiloxane complex were added, and then 33.18 g of methylhydrogenpolysiloxane expressed by the following general formula was added dropwise while maintaining a temperature range of 70°C to 80°C.

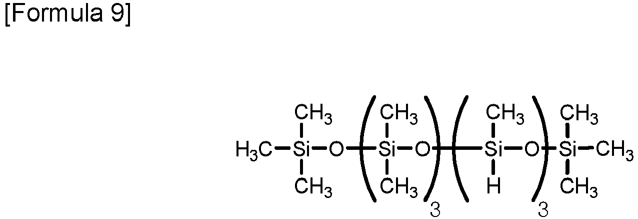

After dropping was complete, the mixture was cured for 3 hours at 110°C, and then the completion of the reaction was confirmed by a hydrogen generation method. The low boiling point content was distilled off under reduced pressure. Thereafter, 20 g of ion exchanged water was added, curing was performed with reflux for 4 hours, and deprotection was performed. Thereafter, the low boiling point content was again removed under reduced pressure to obtain compound 2. As a result of the analysis, it was confirmed that the product was compound 2 expressed by the following chemical structural formula.

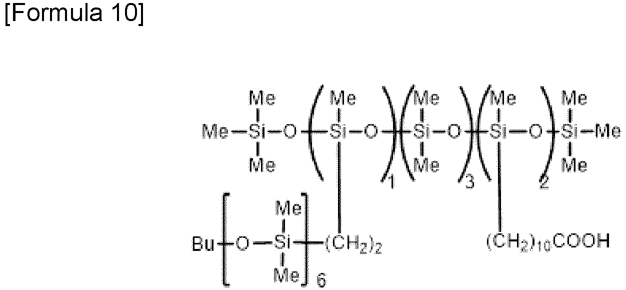

### [Example 3]

In a flask equipped with a stirrer, a reflux condenser, a dropping funnel, and a thermometer, 235.52 g of trimethylsilyl undecylenate, 45.92 g of tristrimethylsiloxyvinylsilane, and 0.078 g of platinum-1,3-divinyl-1,1,3,3-tetramethyldisiloxane complex were added, and then 68.57 g of methylhydrogenpolysiloxane expressed by the following general formula was added dropwise while maintaining a temperature range of 70°C to 80°C.

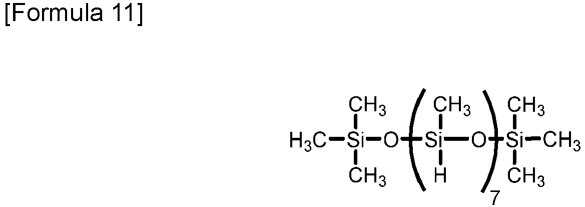

After dropping was complete, the mixture was cured for 3 hours at 110°C, and then the completion of the reaction was confirmed by a hydrogen generation method. The low boiling point content was distilled off under reduced pressure. Thereafter, 90 g of ion exchanged water was added, curing was performed with reflux for 4 hours, and deprotection was performed. Thereafter, the low boiling point content was again removed under reduced pressure to obtain compound 3. As a result of the analysis, it was confirmed that the product was compound 3 expressed by the following chemical structural formula.

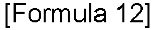

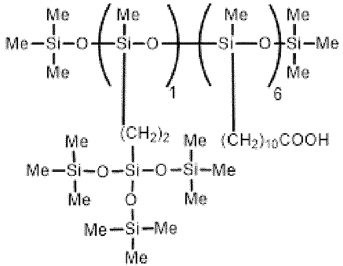

### [Example 4]

In a flask equipped with a stirrer, a reflux condenser, a dropping funnel, and a thermometer, 120.58 g of trimethylsilyl undecylenate, 44.24 g of a macromonomer raw material expressed by Bu-(OSiMe₂)₆-CH=CH₂, and 0.044 g of platinum-1,3-divinyl-1,1,3,3-tetramethyldisiloxane complex were added, and then 35.18 g of methylhydrogenpolysiloxane expressed by the following general formula was added dropwise while maintaining a temperature range of 70°C to 80°C.

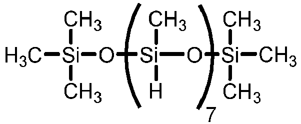

After dropping was complete, the mixture was cured for 3 hours at 110°C, and then the completion of the reaction was confirmed by a hydrogen generation method. The low boiling point content was distilled off under reduced pressure. Thereafter, 50 g of ion exchanged water was added, curing was performed with reflux for 4 hours, and deprotection was performed. Thereafter, the low boiling point content was again removed under reduced pressure to obtain compound 4. As a result of the analysis, it was confirmed that the product was compound 4 expressed by the following chemical structural formula.

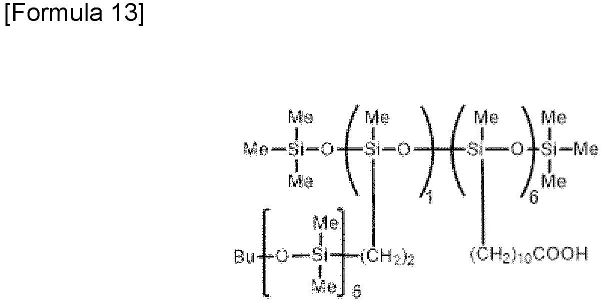

The carboxylic acid-modified silicone (comparative compound 1 to comparative compound 3) used in the comparative example, was synthesized by the following method.

### [Comparative Synthesis Example 1]

In a flask equipped with a stirrer, a reflux condenser, a dropping funnel, and a thermometer, 230.67 g of trimethylsilyl undecylenate and 0.042 g of platinum-1,3-divinyl-1,1,3,3-tetramethyldisiloxane complex were added, and then 129.33 g of methylhydrogenpolysiloxane expressed by the following general formula was added dropwise while keeping a temperature range of 70°C to 80°C.

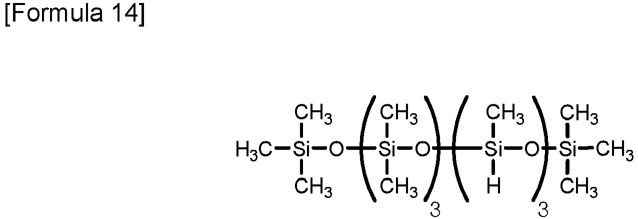

After dropping was complete, the mixture was cured for 2 hours at 110°C, and then completion of the reaction was confirmed by a hydrogen generation method. The low boiling point content was distilled off under reduced pressure. Thereafter, 90 g of ion exchanged water was added, curing was performed with reflux for 4 hours, and deprotection was performed. Thereafter, the low boiling point content was again removed under reduced pressure to obtain comparative compound 1. As a result of the analysis, it was confirmed that the product was comparative compound 1 expressed by the following chemical structural formula.

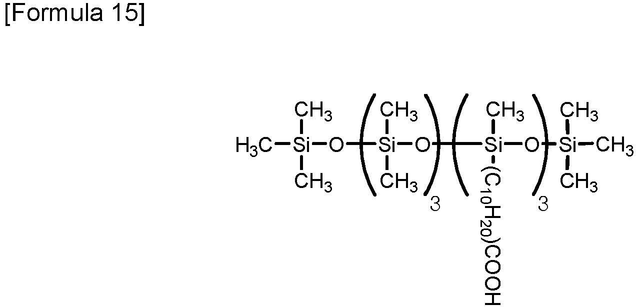

### [Comparative Synthesis Example 2]

In a flask equipped with a stirrer, a reflux condenser, a dropping funnel, and a thermometer, 100 g of 1,1,1,3,5,5,5-heptamethyltrisiloxane, and 0.02 g of toluene solution of platinum-1,3-divinyl-1,1,3,3-tetramethyldisiloxane complex were added, and 105 g of trimethylsilyl undecylenate was added dropwise while keeping a temperature range of 70°C to 100°C. After dropping was complete, the mixture was cured for 2 hours at 110°C, and then completion of the reaction was confirmed by a hydrogen generation method. The low boiling point content was distilled off under reduced pressure. Thereafter, water was added, aged at reflux for 4 hours, and deprotection was performed. Thereafter, the low boiling point content was again removed under reduced pressure to obtain comparative compound 2. As a result of the analysis, it was confirmed that the chemical structure of the comparative compound 2 can be expressed by the following chemical formula.

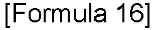

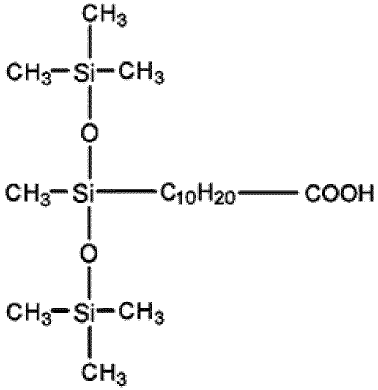

### [Comparative Synthesis Example 3]

In a flask equipped with a stirrer, a reflux condenser, a dropping funnel, and a thermometer, 581.78 g of trimethylsilyl undecylenate and 0.087 g of platinum-1,3-divinyl-1,1,3,3-tetramethyldisiloxane complex were added, and then 168.23 g of methylhydrogenpolysiloxane expressed by the following general formula was added dropwise while keeping a temperature range of 70°C to 80°C.

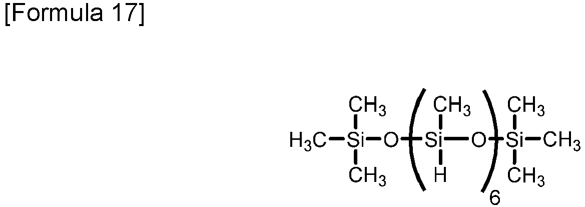

After dropping was complete, the mixture was cured for 3 hours at 110°C, and then completion of the reaction was confirmed by a hydrogen generation method. The low boiling point content was distilled off under reduced pressure. Thereafter, 190 g of ion exchanged water was added, curing was performed with reflux for 4 hours, and deprotection was performed. Thereafter, the low boiling point content was again removed under reduced pressure to obtain comparative compound 3. As a result of the analysis, it was confirmed that the product was comparative compound 3 expressed by the following chemical structural formula.

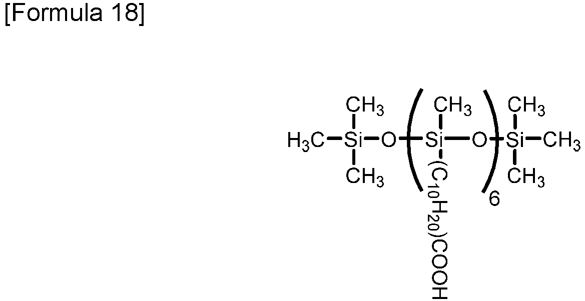

### [Comparative Synthesis Example 4]

In a flask equipped with a stirrer, a reflux condenser, a dropping funnel, and a thermometer, 304.20 g of trimethylsilyl undecylenate and 0.092 g of platinum-1,3-divinyl-1,1,3,3-tetramethyldisiloxane complex were added, and then 304.20 g of methylhydrogenpolysiloxane expressed by the following general formula was added dropwise while keeping a temperature range of 70°C to 80°C.

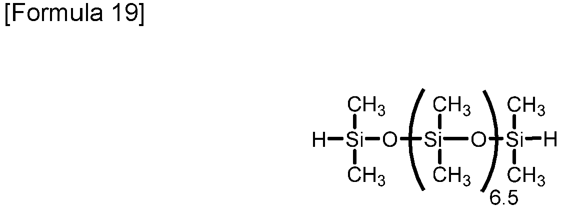

After dropping was complete, the mixture was cured for 3 hours at 110°C, and then completion of the reaction was confirmed by a hydrogen generation method. The low boiling point content was distilled off under reduced pressure. Thereafter, 171.6 g of methanol was added, curing was performed with reflux for 4 hours, and deprotection was performed. Thereafter, the low boiling point content was again removed under reduced pressure to obtain comparative compound 4. As a result of the analysis, it was confirmed that the product was comparative compound 4 expressed by the following chemical structural formula.

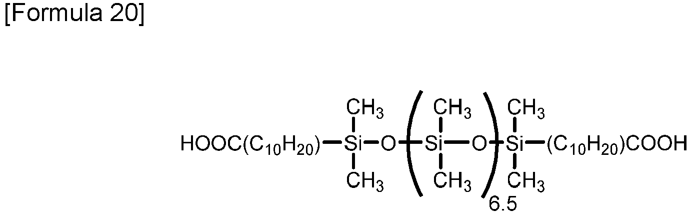

### [Preparation of aqueous dispersion: Examples 5 to 8, Comparative Examples 1 to 12]

The components shown in Table 1 and Table 2 below were put into a mayonnaise bottle, and 160 g of zirconia beads (YTZ ball, ϕ0.8 mm) were added thereinto. After dispersing with a paint shaker and separating the beads, aqueous dispersions of Example 5 to 6 and Comparative Example 1 to 5 were obtained. The aqueous dispersions were evaluated by the following methods, and the results are shown in Table 1. Note that the viscosity of each aqueous dispersion was measured using an EMD viscometer (made by Tokyo Keiki) immediately after preparation (initial), after 4 days of standing at room temperature, and after 4 months of standing at room temperature, and evaluated for 5 levels using the following criteria. Note that if the dispersion was not stable and phase separation occurred, the sample was evaluated as "separated".

### [Appearance evaluation for aqueous dispersion]

The appearance of the aqueous dispersion was observed immediately after preparation.
O: Dispersion was good, and low-viscosity dispersion was obtained
X: Dispersion was poor and it became creamy

### [Evaluation of aqueous dispersion after standing for 4 months]

1: Highly unsuitable as a cosmetic raw material
2: Unsuitable as a cosmetic raw material
3: Usable as a cosmetic raw material
4: Useful as a cosmetic raw material
5: Very useful as a cosmetic raw material

**[Table 1]**

| Component (g) | **Embodiments** | | Comparative Examples | | | | | |
|---|---|---|---|---|---|---|---|---|
| | 5 | **6** | **1** | **2** | 3 | 4 | 5 | 6 |
| A1) Carboxylic acid co-modified organopolysiloxane (Compound 3) | 2 | 2 | | | | | | |
| A) Carboxylic acid-modified silicone (Comparative compound 1) | | | 2 | 2 | 2 | | | |
| A") Carboxylic acid-modified silicone (Comparative compound 2) | | | | | | 2 | | |
| A") Carboxylic acid-modified silicone (Comparative compound 3) | | | | | | | 2 | |
| Aʺʺ) Isostearic acid (Note 1) | | | | | | | | 2 |
| C1) Hydrophobic titanium dioxide (Note 2) Average particle size 10 × 90 nm | 12 | 12 | 12 | 12 | 12 | 12 | 12 | 12 |
| B) Aminomethylpropanol | 0.58 | 0.58 | 0.57 | 0.57 | 0.57 | 0.47 | 0.71 | 0.67 |
| (neutralizing agent) | | | | | | | | |
| D1) Ion exchanged water | 16 | 16 | 26 | 16 | 16 | 16 | 16 | 16 |
| D2) Ethyl alcohol | | 10 | | | 10 | 10 | 10 | 10 |
| D3) 1,3-butanediol | 10 | | | 10 | | | | |
| Amount of COOH (KOH mg/g) | 171 | 171 | 168 | 168 | 168 | 138 | 207 | 197 |
| Appearance of aqueous dispersion | ○ | ○ | ○ | ○ | × | ○ | × | × |
| Viscosity of aqueous dispersion (initial) | 24 | 54 | 46 | 27 | 313 | 58 | 360 | 363 |
| mPa·s | | | | | | | | |
| Viscosity of aqueous dispersion (after 4 days at room temperature) | 23 | 74 | 29 | 26 | 353 | 71 | * | * |
| mPa·s | | | | | | | | |
| Stability evaluation of aqueous dispersion (After 4 months at room temperature) | 5 | 3 | 4 | 5 | 2 | 4 | 1 | 1 |

| | | | | | | | | |
|---|---|---|---|---|---|---|---|---|
| (Note 1) Product name: Isostearic acid EX (available from Kokyu Alcohol Kogyo Co., Ltd.) (Note 2) Product name: STR-100A-LP (manufactured by Sakai Chemical Industry Co., Ltd.) *Measurement not possible due to high viscosity | | | | | | | | |

**[Table 2]**

| Component (g) | **Embodiments** | | Comparative Examples | | | | | |
|---|---|---|---|---|---|---|---|---|
| | 7 | **8** | **7** | **8** | 9 | 10 | 11 | 12 |
| A1) Carboxylic acid co-modified organopolysiloxane (Compound 3) | 2 | | | | | | | |
| A2) Carboxylic acid co-modified organopolysiloxane (Compound 4) | | 2 | | | | | | |
| A') Carboxylic acid-modified silicone (Comparative compound 1) | | | 2 | 2 | | | | |
| A") Carboxylic acid-modified silicone (Comparative compound 2) | | | | | | 2 | | |
| A‴) Carboxylic acid-modified silicone (Comparative compound 3) | | | | | | | 2 | |
| Aʺʺ) Isostearic acid (Note 1) | | | | | | | | 2 |
| Aʺ‴) Carboxylic acid-modified silicone (Comparative compound 4) | | | | | 2 | | | |
| C1) Hydrophobic zinc oxide (Note 3) Average particle size 35 nm | 12 | 12 | 12 | 12 | 12 | 12 | 12 | 12 |
| B) Aminomethylpropanol (neutralizing agent) | 0.58 | 0.53 | 0.57 | 0.57 | 0.39 | 0.47 | 0.71 | 0.67 |
| D1) Ion exchanged water | 16 | 16 | 26 | 16 | 16 | 16 | 16 | 16 |
| D2) Ethyl alcohol | 10 | 10 | | 10 | 10 | 10 | 10 | 10 |
| Amount of COOH (KOH mg/g) | 171 | 155 | 168 | 168 | 168 | 138 | 207 | 197 |
| Appearance of aqueous dispersion | ○ | ○ | ○ | ○ | ○ | ○ | × | × |
| Viscosity of aqueous dispersion (initial) mPa·s | 10 | 45 | 4 | 31 | 66 | 19 | * | 24 |
| Viscosity of aqueous dispersion (after 4 days at room temperature) | 13 | 120 | Separated | Separated | Separated | Separated | * | Separated |
| mPa·s | | | | | | | | |
| Stability evaluation of aqueous dispersion | 5 | 3 | 1 | 1 | 1 | 1 | 1 | 1 |
| (After 4 months at room temperature) | | | | | | | | |

| | | | | | | | | |
|---|---|---|---|---|---|---|---|---|
| (Note 1) Product name: Isostearic acid EX (available from Kokyu Alcohol Kogyo Co., Ltd.) (Note 3) Product name: FINEX-30S-LPT (available from Sakai Chemical Industry Co., Ltd.) *Measurement not possible due to high viscosity | | | | | | | | |

As shown in Tables 1 and 2, the carboxylic acid co-modified organopolysiloxane according to the examples were used to obtain powder compositions for hydrophobic titanium dioxide and hydrophobic zinc oxide fine particles, which are low viscosity aqueous slurries (aqueous dispersions) with these powders stably dispersed in the aqueous phase. These aqueous slurries are useful as cosmetic raw materials or the like. In addition, by using the carboxylic acid co-modified organopolysiloxane according to the Examples, it is anticipated that topical agent compositions such as sunscreen cosmetic materials in which these powders are stably dispersed in the aqueous phase can be easily designed.

On the other hand, the carboxylic acid-modified silicones and higher fatty acids according to the comparative examples provided a powder composition which is a low-viscosity aqueous slurry (aqueous dispersion) with a certain level of stability with respect to hydrophobic titanium dioxide, but with respect to hydrophobic zinc oxide fine particles, the aqueous slurries (aqueous dispersions) were observed to rapidly thicken or undergo phase separation immediately after preparation for four days, and thus rapid thickening and phase separation were observed. Therefore, with the known carboxylic acid-modified silicones and higher fatty acids according to the comparative examples, sufficient dispersion performance cannot be achieved for some hydrophobic fine particles, and stable aqueous slurries and topical agent compositions such as sunscreen cosmetic materials may not be achieved. Thus, there is insufficient freedom in formulation design, and there is concern that the stability of the resulting cosmetic raw materials and the performance of cosmetic materials and other topical agent compositions may be insufficient.

### [Example of formulation design and formulation]

The carboxylic acid co-modified organopolysiloxanes according to the present invention can be used to replace some or all of the carboxylic acid-modified silicone in formulation examples of topical agent compositions for known carboxylic acid-modified silicones. Specifically, the carboxylic acid-modified silicones according to the examples and formulation examples disclosed in Patent Document 1 (International Published Patent WO2015/159773), Patent Document 2 (International Published Patent WO2020/036065), International Patent Applications (PCT/JP19/029957, PCT/JP19/029927), International Published Patent WO/2020/036064, International Publication Patent WO/2020/036061, International Publication Patent WO/2020/036062, and International Publication Patent WO/2020/036063 being replaced by the carboxylic acid co-modified organopolysiloxane (compound 1 to 4) according to examples 1 to 4 is included in the scope of the present invention as a formulation example of the cosmetic material according to the present invention, and thus the present applicant explicitly teaches this type of use and formulation design.

## Claims

1. A carboxylic acid co-modified organopolysiloxane expressed by the following structural formula (1), which is a liquid at 50°C. 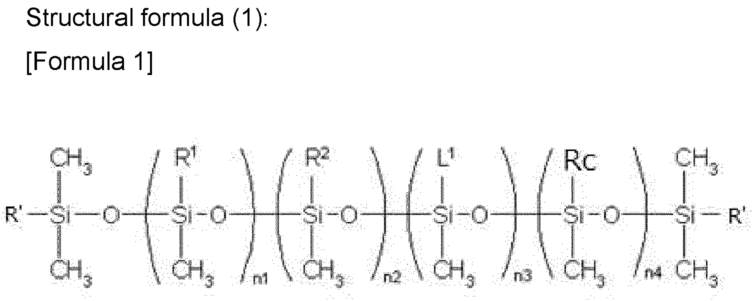 {where,
R¹ is a substituted or unsubstituted monovalent hydrocarbon group having 1 to 6 carbon atoms, or a hydrogen atom; R² represents a substituted or unsubstituted straight-chain or branched monovalent hydrocarbon group having 6 to 30 carbon atoms;
L¹ represents one or more type of group selected from:
a silylalkyl group having the siloxane dendron structure expressed by the following general formula (2) when i = 1: 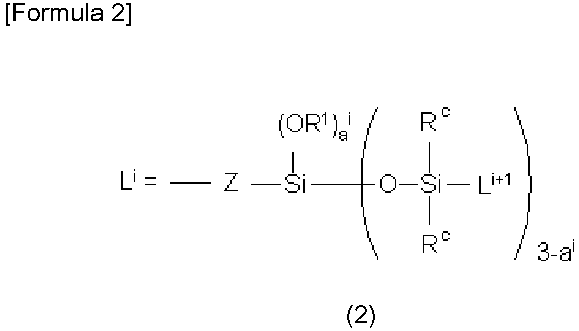
(where R¹ is a group similar to the groups described above, R^{C} is an alkyl group with 1 to 6 carbon atoms, or a phenyl group, and Z is a bivalent organic group. i in Lⁱ represents the number of units of silylalkyl groups expressed by Lⁱ and is an integer from 1 to c, where c represents the number of units, or in other words the number of repetitions of silylalkyl groups, and where the number of units c is an integer from 1 to 10. Lⁱ⁺¹ represents a silylalkyl group where i is less than c and is a methyl group or a phenyl group when i=c. aⁱ is a number from 0 to 3);
a chain organosiloxane group, expressed by the following general formula (2'): 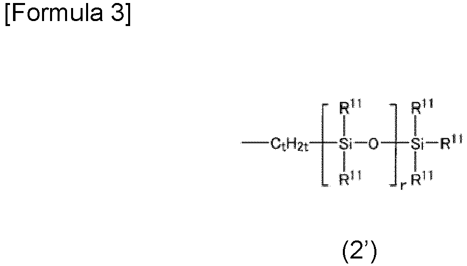
(where R¹¹ are each independently a substituted or unsubstituted monovalent hydrocarbon group having 1 to 30 carbon atoms, hydroxyl group, or hydrogen atom, and at least one of the R¹¹ groups is a monovalent hydrocarbon group; t is a number in a range of 2 to 10; and r is a number in a range of 1 to 500); and
a chain type organosiloxane group expressed by the following general formula (2"): 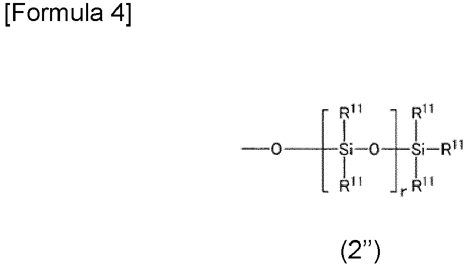
(where R¹¹ and r are similar to those described above);
Rc represents a carboxyl group-containing organic group expressed by a general formula: -Rc¹-(ORc²)p-(O)w-Rc³-COOH, (Rc¹ represents a straight-chain or branched alkylene group having 2 to 22 carbon atoms, R² represents a straight-chained or branched alkylene group having 2 to 4 carbon atoms, R³ represents a bond (-) or a straight-chain or branched alkylene group having 1 to 22 carbon atoms, p represents a number from 0 to 200, and w represents a number that is 0 or 1),
R' is a group independently selected from R¹, R², L¹ and Rc described above, and
(n1 + n2 + n3 + n4) is a number in a range of 1 to 1000; n1 is a number in a range of 0 to 999; n2 is a number in a range of 0 to 100; n3 is a number in a range of 1 to 100; and n4 is a number in a range of 0 to 100; and if n4 = 0, at least one R' is Rc.}

2. The carboxylic acid co-modified organopolysiloxane according to claim 1, wherein in formula (1), L¹ is a functional group expressed by the following general formula (2-1), general formula (2-2) or general formula (2'). 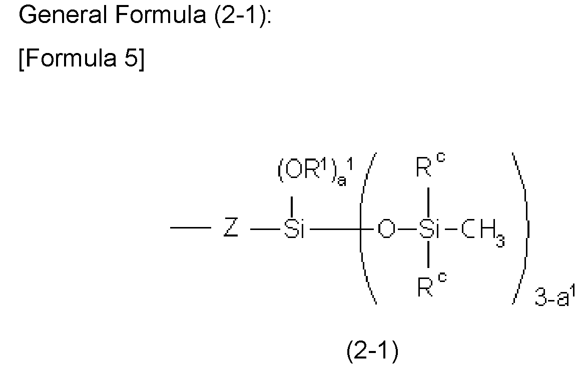 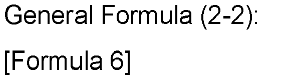 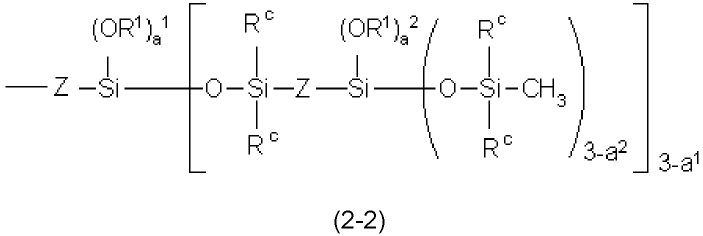
(where R¹, R^{C}, and Z are similar to the groups described above, and a¹ and a² are both independently a number in a range of 0 to 3). 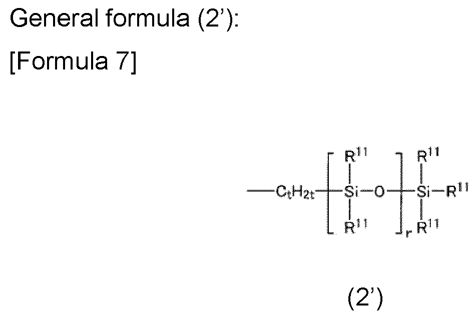
(In the formula, R¹¹ is independently similar to the groups described above, t is a number in a range of 2 to 10, and r is a number in the range of 1 to 500).

3. The carboxylic acid co-modified organopolysiloxane according to claim 1 or claim 2, wherein, in the above structural formula (1), (n1 + n2 + n3 + n4) is a number in the range of 2 to 50, n1 is 0 to 49, n2 is 0 to 10, n3 is 1 to 10, n4 is 0 to 10, and when n4 = 0, at least one of R' is Rc.

4. The carboxylic acid co-modified organopolysiloxane according to any one of claims 1 to 3, that is a liquid at room temperature (25°C), and in the above structural formula (1), (n1 + n2 + n3 + n4) is a number in a range 2 to 20, n3 is a number in a range 1 to 10, n4 is a number in a range 1 to 10, and (n1 + n2 + n3)/n4 is in a range 0.1 to 5.0.

5. The carboxylic acid co-modified organopolysiloxane composition, comprising:
(A) the carboxylic acid co-modified organopolysiloxane according to any one of claims 1 to 4;
(B) a basic compound; and
(C) one or more hydrophilic mediums selected from water, polyhydric alcohols, and lower alcohols.

6. A powder treatment agent, comprising: a carboxylic acid co-modified organopolysiloxane according to any one of claims 1 to 4, or a carboxylic acid co-modified organopolysiloxane composition according to claim 5.

7. A surfactant or surface treatment agent, comprising: a carboxylic acid co-modified organopolysiloxane according to any one of claims 1 to 4 or a carboxylic acid co-modified organopolysiloxane composition according to claim 5.

8. A powder composition, comprising:
(A) the carboxylic acid co-modified organopolysiloxane according to any one of claims 1 to 4;
(B) a basic compound;
(C) one or more hydrophilic mediums selected from water, polyhydric alcohols and lower alcohols; and
(D) a powder or colorant.

9. A powder composition according to claim 8, wherein the aforementioned component (D) is (D1) a hydrophobic powder or colorant, and component (D) has a structure dispersed in an aqueous phase.

10. The powder composition according to claim 8, wherein the aforementioned component (D) is (D1-1) hydrophobized fine particle titanium dioxide or hydrophobized fine particle zinc oxide with an average particle size in a range of 1 to 200 nm, and component (D) has a structure that is dispersed in the aqueous phase.

11. A topical agent composition, comprising: a carboxylic acid co-modified organopolysiloxane according to any one of claims 1 to 4, or a carboxylic acid co-modified organopolysiloxane composition according to claim 5.

12. A topical agent composition, comprising:
(A) the carboxylic acid co-modified organopolysiloxane according to any one of claims 1 to 4;
(B) a basic compound;
(C) one or more hydrophilic mediums selected from water, polyhydric alcohols and lower alcohols; and
(D) a powder or colorant;
wherein component (D) is dispersed in the aqueous phase.

13. A topical agent composition, comprising: the powder composition according to any one of claims 8 to 10.

14. The topical agent composition according to any one of claims 11 to 13, that is a cosmetic material or a medicament.

15. A method for manufacturing a topical agent composition according to any one of claims 12 to 14, comprising a step of premixing:
(A) the carboxylic acid co-modified organopolysiloxane according to any one of claims 1 to 4;
(B) a basic compound;
(C) one or more hydrophilic mediums selected from water, polyhydric alcohols and lower alcohols; and
(D) a powder or colorant;
wherein component (D) is added in the form of an aqueous dispersion.
